# EUROPÄISCHE PATENTANMELDUNG

(11) **EP 1 661 553 A1**
(43) Veröffentlichungstag der Anmeldung: **31.05.2006**
(21) Anmeldenummer: 05105598.6
(22) Anmeldetag: 23.06.2005
(51) Int. Cl.: A61K 8/81, A61K 8/73, A61K 8/86, A61K 8/04, A61Q 5/06

(54) **Mittel zur fixierenden Behandlung keratinischer Fasern mit einer Kombination aus fixierenden Polymeren mit bestimmten Molmassen**

(30) Priorität: 25.08.2004 DE 102004041294
(71) Anmelder: Beiersdorf AG, 20245 Hamburg (DE)
(72) Erfinder: DETERT, Marion, 22455, Hamburg (DE); SASS, Viola, 25488, Holm (DE)

(57) **Zusammenfassung**

Zubereitung zur Fixierung keratinischer Fasern umfassend
a) 0,05 - 10% (bevorzugt 0,1 - 5%) eines kationischen Polymers mit einer mittleren Molmasse von 80.000 - 120.000, bevorzugt 100.000,
b) 0,05 - 10% (bevorzugt 0,1 - 5%) eines kationischen Polymers mit einer mittleren Molmasse von 300.000 - 500.000, bevorzugt 400.000,
c) 0 - 10% eines kationischen Polymers mit einem mittleren Molmasse von 800.000 - 1200.000, bevorzugt 1000.000,
d) 0,05 - 15% (bevorzugt (0,1 - 8 %) eines nichtionischen Polymers aufgebaut aus Vinyllactammonomeren,
e) 0,05 - 5% (bevorzugt 0,01 - 1%) eines amphoteren, nichtionischen, anionischen oder kationischen Tensid.

## Beschreibung

Die vorliegende Erfindung betrifft Mittel zur fixierenden Behandlung keratinischer Fasern mit einer Kombination aus fixierenden Polymeren mit bestimmten Molmassen, insbesondere Schaumfestiger zur Anwendung auf dem Haar.

Eine Zubereitung zur Fixierung keratinischer Fasern im Sinne der vorliegenden Schrift kann ein Haarfestiger, auch Festigerschaum oder Schaumfestiger, aber auch eine nicht schäumende oder nicht aufgeschäumte Zubereitung sein.

Kationische Polymere im Sinne der vorliegenden Schrift sind makromolekulare Substanzen, die mehr positive als negative Ladungen im Molekül selbst, d.h. ohne Berücksichtigung von Gegenionen, aufweisen.

Die mittlere Molmasse im Sinne der vorliegenden Schrift stellt das Gewichtsmittel der Molmassen dar.

Prozent (%) im Sinne der vorliegenden Schrift sind Gewichtsprozent.

Zur temporären Verformung und Stabilisierung der Frisur werden Produkte eingesetzt, die als Stylingmittel bekannt sind. Hierzu zählen u.a. auch Haarsprays, Haarfestiger oder Festigerschäume. Haarfestiger werden als Aerosol (Schaumfestiger), sogenannte Pumpfoamer oder Sprühfestiger angeboten.

Üblicherweise bestehen diese Mittel zur Festigung der Haare aus alkoholischen oder wässrig alkoholischen Lösungen von filmbildenden natürlichen oder synthetischen Polymeren, auch als fixierende Polymere bezeichnet. Als solche Polymere werde oft nichtionische, amphotere, anionische oder kationische Polymere eingesetzt, die für die Halteeigenschaften verantwortlich sind. Gerade bei Haarfestigern, insbesondere bei schäumenden Produkten sind die pflegenden Eigenschaften, wie z.B. gute Kämmbarkeit der Haare im nassen und trocknen Zustand, der Griff der Haare im nassen und trocknen Zustand wichtige Kriterien, die einen "gepflegten" Zustand der Haare charakterisieren. Außerdem ist wichtig, dass die Haare nicht beschwert werden, dafür die Frisur mit sichtbaren Volumen versorgt wird.

Der Einsatz von festigenden Polymeren fixiert die erstellte Frisur, lässt das Haar aber eher belegt und rau erscheinen. Der Griff der Haare ist nicht mehr glatt und gepflegt. Daher sind die pflegenden Eigenschaften bei herkömmlichen Haarfestigern noch nicht optimal. Der Zusatz von weiteren konditionierenden Rohstoffen führt in der Regel zu einer Beschwerung der Haare und somit zu einer Reduzierung des Volumens.

WO 8908443 betrifft Haarpflegezubereitungen mit kationischem Vinylpyrrolidon-Vinylimidazolium methochlorid-Copolymer mit einem Molgewicht von 40000 bis 1000000 g/mol und Tetraoxyethylenlauryl ether.

EP 1340485 betrifft schäumende Zubereitungen mit Treibgas und flüssiger Phase enthaltend Tensid, kationische Vinylpyrrolidonpolymere und Zelluloseverbindungen.

Es hat sich für den Fachmann nicht vorhersehbar herausgestellt, dass eine Zubereitung zur Fixierung keratinischer Fasern umfassend
a) 0,05 - 10%, bevorzugt 0,1 - 5% eines kationischen Polymers mit einer mittleren Molmasse von 80.000 - 120.000, bevorzugt 100.000,
b) 0,05 - 10%, bevorzugt 0,1 - 5% eines kationischen Polymers mit einer mittleren Molmasse von 300.000 - 500.000, bevorzugt 400.000,
c) 0 - 10% eines kationischen Polymers mit einem mittleren Molmasse von 800.000 - 1200.000, bevorzugt 1000.000,
d) 0,05 - 15%, bevorzugt 0,1 - 8 % eines nichtionischen Polymers aufgebaut aus Vinyllactammonomeren,
e) 0,05 - 5%, bevorzugt 0,1 - 1% eines amphoteren, nichtionischen, anionischen oder kationischen Tensids den Mängeln des Standes der Technik abhelfen, indem sie die negativen Eigenschaften üblicher Festiger verbessern. Das Produkt wird als Schaum auf die Haare aufgetragen. Die erfindungsgemäße Kombination führt dazu, dass der Haarfestiger neben den guten Halteeigenschaften das Haar mit einem guten Griff und einer guten Kämmbarkeit versorgt. Das Haar wirkt nicht mehr belegt sondern gepflegt. Ihm kommt somit eine überragende pflegende Leistung zu, wobei die Stylingeigenschaften des Produktes nicht negativ beeinträchtigt werden. Außerdem sind die Haare nicht zu beschwert was sich in einem verbesserten Volumen zeigt.

Es wurde weiter gefunden, dass es bevorzugt ist, wenn das kationische Polymer mit einer mittleren Molmasse von 80.000 - 120.000, bevorzugt um 100.000 aus der Gruppe der Methvinylimidazoliumchlorid/Vinylpyrrolidon Copolymer ausgewählt wird. Weiter bevorzugt ist es, wenn das kationisches Polymer mit einer mittleren Molmasse von 300.000 - 500.000, bevorzugt um 400.000 aus der Gruppe der modifizierten quaternisierten Stärkederivate ausgewählt wird. Darüber hinaus ist es bevorzugt, wenn das kationisches Polymer mit einer mittleren Molmasse von 700.000 - 1200.000, bevorzugt um 1000.000 aus der Gruppe der quaternisierten Vinylpyrrolidon/Dimethylaminoethylmethacrylat Copolymere ausgewählt wird. Dabei ist es bevorzugt, wenn das Tensid ein nichtionisches Tensid ist. Ebenfalls bevorzugt ist es, wenn zusätzlich bis 20%, besonders bevorzugt 4 bis 11 % Treibmittel enthalten sind.

Besonders bevorzugt ist es, wenn das kationische Polymer mit einer mittleren Molmasse von 80.000 - 120.000, bevorzugt um 100.000 PQ-16 ist, wenn das kationisches Polymer mit einer mittleren Molmasse von 300.000 - 500.000, bevorzugt um 400.000 PQ-4 ist und/oder wenn das kationisches Polymer mit einer mittleren Molmasse von 700.000 - 1200.000, bevorzugt um 1000.000 PQ-11 ist. Darüber hinaus ist es besonders bevorzugt, wenn das nichtionische Polymer VP/VA Copolymer ist und/oder das Tensid einen HLB Wert größer als 12 besitzt oder ganz besonders bevorzugt PEG-40 Hydrogenated Castor Oil ist. Die Erfindung umfasst auch die Verwendung einer solchen als Haarfestiger.

Als Copolymer aus Vinylpyrrolidon und quaternisierten Vinylimidazol im Sinne der vorliegenden Erfindung können die Produkte Luviquat FC 370 (Fa. BASF), Luviquat FC 550 (Fa. BASF), Luviquat HM 552 (Fa. BASF), Luviquat FC 905 (Fa. BASF), Luviquat Hold (Fa. BASF) oder Luviquat MS 370 (Fa. BASF) eingesetzt werden.

Als modifiziertes quaternisiertes Stärkederivate im Sinne der vorliegenden Erfindung kann PQ-4 eingesetzt werden, wie z.B. Celquat L-200 oder Celquat H-100 von National Starch.

Als quaternisierte Vinylpyrrolidon/Dimethylaminoethylmethacrylat Copolymere im Sinne der vorliegenden Erfindung können Copolymer 845 (Fa. ISP), Copolymer 937 (Fa. ISP), Copolymer 958 (Fa. ISP) oder Gafquat 755N (Fa. ISP) eingesetzt werden.

Als nichtionische Tenside im Sinne der vorliegenden Erfindung können Fettalkoholethoxylate, Fettsäureethoxylate, Fettsäuremonoglyceride, Fettsäureglycolpartialester oder Polyoxyethylenglycolfettsäureester eingesetzt werden.

Vorteilhaft ist die Verwendung von nichtionischen Tensiden mit einem HLB Wert der größer als 12 ist, bevorzugt 13 ― 16.

Als Treibmittel im Sinne der vorliegenden Erfindung können Dimethylether oder Kohlenwasserstofftreibgase wie z.B. n-Butan, i-Butan, Propan sowie komprimierte Gase wie N₂ , N₂ O, CO₂ eingesetzt werden.

Als kationische Polymere mit einer mittleren Molmasse von 80.000 - 120.000, bevorzugt um 100.00 0im Sinne der vorliegenden Erfindung können bevorzugt Luviquat FC 370 (INCI: Polyquaternium-16) von Fa. BASF eingesetzt werden.

Als kationisches Polymer mit einer mittleren Molmasse von 300.000 - 500.000, bevorzugt
400.000 im Sinne der vorliegenden Erfindung können bevorzugt Celquat I-200 (INCI: Polyquaternium-4) von Fa. National Starch eingesetzt werden.

Als kationisches Polymer mit einer mittleren Molmasse von 700.000 - 1200.000, bevorzugt 1000.000 im Sinne der vorliegenden Erfindung können bevorzugt Gafquat 755N (INCI: Polyquaternium-11) von Fa. ISP, eingesetzt werden.

Als nichtionisches Tensid wird bevorzugt Polyethylenglycol hydriertes Rizinusöl eingesetzt z.B. Cremophor RH 410 (INCI: PEG 40-Hydrogenated Castor Oil) von der Fa. BASF.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Die erfindungsgemäßen Zubereitungen können ferner Wasser, Alkohole, übliche Siliconöle, übliche kationische Tenside, übliche Neutralisationsmittel enthalten.

Optionale herkömmliche Additive können ebenfalls in die Formulierung einbezogen sein, um der Zusammensetzung bestimmte Modifizierungseigenschaften zu verleihen: dies sind Silikone oder Silikonderivate, Benetzungsmittel, Feuchthaltemittel, Weichmacher wie Glycerin, Glykol und Phthalester und - ether, erweichende Mittel, Riechstoffe und Parfüms, UV- Absorber, Farbstoffe , Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, oberflächenaktive Mittel, Viskositätsmodifizierer, Verdicker, Geliermittel, Trübungsmittel, Stabilisatoren, Sequestierungsmittel, Komplexbildner, Perlglanzmittel, ästhetische Verstärker, botanische Extrakte und Klärhilfsmittel.

Diese Additive sind im allgemeinen in einer Konzentration von etwa 0,01% bis 10 Gew.% bezogen auf das Gesamtgewicht der Zusammensetzung vorhanden.

Das erfindungsgemäße Mittel enthält anionische, kationische, amphotere oder nichtionische Tenside: Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielsweise ―COO⁻, ―OSO₃²⁻, ―SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische lonen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:
RNH₂⁺CH₂CH₂COOH X⁻ (bei pH=2) X⁻ = beliebiges Anion, z.B. Cl⁻
RNH₂⁺CH₂CH₂COO⁻ (bei pH=7)
RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wässrigem Medium keine Ionen.
A. Anionische Tenside
   Vorteilhaft zu verwendende anionische Tenside sind
   Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. Acyllactylate, Lauroyllactylat, Caproyllactylat
   6. Alaninate
   Carbonsäuren und Derivate, wie
   1. Carbonsäuren, beispielsweise Laurinsäure, Aluminiumstearat, Magnesiumalkanolat und Zinkundecylenat,
   2. Ester-Carbonsäuren, beispielsweise Calciumstearoyllactylat, Laureth-6-Citrat und Natrium PEG-4-Lauramidcarboxylat,
   3. Ether-Carbonsäuren, beispielsweise Natriumlaureth-13-Carboxylat und Natrium PEG-6-Cocamide Carboxylat,
   Phosphorsäureester und Salze, wie beispielsweise DEA-Oleth-10-Phosphat und Dilaureth-4 Phosphat,
   Sulfonsäuren und Salze, wie
   1. Acyl-isethionate, z.B. Natrium-/ Ammoniumcocoyl-isethionat,
   2. Alkylarylsulfonate,
   3. Alkylsulfonate, beispielsweise Natriumcocosmonoglyceridsulfat, Natrium C₁₂₊₁₄ Olefin-sulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
   sowie
   Schwefelsäureester, wie
   1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
   2. Alkylsulfate, beispielsweise Natrium-, Ammonium- und TEA-Laurylsulfat.
B. Kationische Tenside
   Vorteilhaft zu verwendende kationische Tenside sind
   1. Alkylamine,
   2. Alkylimidazole,
   3. Ethoxylierte Amine und
   4. Quaternäre Tenside.
   5. Esterquats
   Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.
C. Amphotere Tenside
   Vorteilhaft zu verwendende amphotere Tenside sind
   1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
   2. N-Alkylaminosäuren, beispielsweise Aminopropylalkylglutamid, Alkylaminopropionsäure, Natriumalkylimidodipropionat und Lauroamphocarboxyglycinat.
D. Nicht-ionische Tenside
   Vorteilhaft zu verwendende nicht-ionische Tenside sind
   1. Alkohole,
   2. Alkanolamide, wie Cocamide MEA/ DEA/ MIPA,
   3. Aminoxide, wie Cocoamidopropylaminoxid,
   4. Ester, die durch Veresterung von Carbonsäuren mit Ethylenoxid, Glycerin, Sorbitan oder anderen Alkoholen entstehen,
   5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
   6. Sucroseester, -Ether
   7. Polyglycerinester, Diglycerinester, Monoglycerinester
   8. Methylglucosester, Ester von Hydroxysäuren

Als nichtionische Tenside im Sinne der vorliegenden Erfindung können Fettalkoholethoxylate, Fettsäureethoxylate, Fettsäuremonoglyceride, Fettsäureglycolpartialester oder Polyoxyethylenglycolfettsäureeste eingesetzt werden. Vorteilhaft ist die Verwendung von nichtionischen Tensiden mit einem HLB Wert der größer als 12 ist, bevorzugt 13 ― 16.

Bevorzugt eingesetzt wird PEG-40 Hydrogenated Castor Oil wie z.B: Cremophor RH 410 von Fa. BASF oder Sympatens TRH/400 von Fa. Kolb.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen. Die Zahlenwerte in den Beispielen bedeuten Gewichtsprozente, bezogen auf das Gesamtgewicht der jeweiligen Zubereitungen.

### Beispiele

### Beispiele 1 ― 4 - Haarfestiger

| | **1** | **2** | **3** | **4** |
|---|---|---|---|---|
| Polyquaternium-11 | 0,5 | 0,7 | - | - |
| Polyquaternium-16 | 2,0 | 1,0 | 2,0 | 1,0 |
| Polyquaternium-4 | 0,5 | 1,0 | 1,0 | 0,5 |
| VP/VA Copolymer | 3,0 | 4,0 | 5,0 | 5,0 |
| pH Einstellung | q.s. | q.s. | q.s. | q.s. |
| PEG-40 Hydrogenated Castor Oil | - | 0,2 | 0,15 | 0,2 |
| Ceteareth-20 | 0,15 | - | - | - |
| Partum | 0,2 | 0,2 | 0,2 | 0,2 |
| Cetrimoniumchloride | 0,1 | - | 0,1 | 0,1 |
| Dimethicone | - | 0,1 | - | - |
| Ethanol | 10,0 | - | - | 5,0 |
| Treibmittel | 10,0 | 10,0 | 10,0 | 10,0 |
| Wasser | ad 100,0 | ad 100,0 | ad 100 | ad100 |

### Beispiele 5 ― 8 - Haarfestiger

| | **5** | **6** | **7** | **8** |
|---|---|---|---|---|
| Polyquaternium-11 | 0,5 | 0,7 | - | - |
| Polyquaternium-16 | 2,0 | 1,0 | 1,0 | 0,5 |
| Polyquaternium-4 | 0,5 | 1,0 | 1,0 | 0,5 |
| VP/VA Copolymer | 3,0 | 4,0 | 5,0 | 5,0 |
| pH Einstellung | q.s. | q.s. | q.s. | q.s. |
| PEG-40 Hydrogenated Castor Oil | - | 0,2 | 0,15 | 0,2 |
| Ceteareth-20 | 0,15 | - | - | - |
| Parfum | 0,2 | 0,2 | 0,2 | 0,2 |
| Cetrimoniumchloride | 0,1 | - | 0,1 | 0,1 |
| Dimethicone | - | 0,1 | - | - |
| Ethanol | 10,0 | - | - | 5,0 |
| Treibmittel | 10,0 | 10,0 | 10,0 | 10,0 |
| Wasser | ad 100,0 | ad 100,0 | ad 100 | ad100 |

## Patentansprüche

1. Zubereitung zur Fixierung keratinischer Fasern umfassend
a) 0,05 - 10% (bevorzugt 0,1 - 5%) eines kationischen Polymers mit einer mittleren Molmasse von 80.000 - 120.000, bevorzugt 100.000,
b) 0,05 - 10% (bevorzugt 0,1 - 5%) eines kationischen Polymers mit einer mittleren Molmasse von 300.000 - 500.000, bevorzugt 400.000,
c) 0 - 10% eines kationischen Polymers mit einem mittleren Molmasse von 800.000 - 1200.000, bevorzugt 1000.000,
d) 0,05 - 15% (bevorzugt (0,1 - 8 %) eines nichtionischen Polymers aufgebaut aus Vinyllactammonomeren,
e) 0,05 - 5% (bevorzugt 0,01 ― 1%) eines amphoteren, nichtionischen, anionischen oder kationischen Tensid.

2. Zubereitung nach Anspruch 1 **dadurch gekennzeichnet, dass** das kationische Polymer mit einer mittleren Molmasse von 80.000 - 120.000, bevorzugt um 100.000 aus der Gruppe der Methvinylimidazoliumchlorid/Vinylpyrrolidon Copolymer ausgewählt wird.

3. Zubereitung nach einem der vorangehenden Ansprüche **dadurch gekennzeichnet, dass** das kationisches Polymer mit einer mittleren Molmasse von 300.000 - 500.000, bevorzugt um 400.000 aus der Gruppe der modifizierten quaternisierten Stärkederivate ausgewählt wird.

4. Zubereitung nach einem der vorangehenden Ansprüche **dadurch gekennzeichnet, dass** das kationisches Polymer mit einer mittleren Molmasse von 700.000 - 1200.000, bevorzugt um 1000.000 aus der Gruppe der quaternisierten Vinylpyrrolidon/Dimethylaminoethylmethacrylat Copolymere ausgewählt wird.

5. Zubereitung nach einem der vorangehenden Ansprüche **dadurch gekennzeichnet, dass** das Tensid ein nichtionisches Tensid ist..

6. Zubereitung nach einem der vorangehenden Ansprüche **dadurch gekennzeichnet, dass** zusätzlich bis 20%, besonders bevorzugt 4 bis 11 % Treibmittel enthalten sind.

7. Zubereitung nach einem der vorangehenden Ansprüche **dadurch gekennzeichnet, dass** das kationische Polymer mit einer mittleren Molmasse von 80.000 - 120.000, bevorzugt um 100.000 PQ-16 ist.

8. Zubereitung nach einem der vorangehenden Ansprüche **dadurch gekennzeichnet, dass** das kationisches Polymer mit einer mittleren Molmasse von 300.000 - 500.000, bevorzugt um 400.000 PQ-4 ist.

9. Zubereitung nach einem der vorangehenden Ansprüche **dadurch gekennzeichnet, dass** das kationisches Polymer mit einer mittleren Molmasse von 700.000 - 1200.000, bevorzugt um 1000.000 PQ11 ist.

10. Zubereitung nach einem der vorangehenden Ansprüche **dadurch gekennzeichnet, dass** das nichtionische Polymer VP/VA Copolymer ist.

11. Zubereitung nach einem der vorangehenden Ansprüche **dadurch gekennzeichnet, dass** das Tensid einen HLB Wert größer als 12 besitzt oder PEG-40 Hydrogenated Castor Oil ist.

12. Zubereitung nach einem der vorangehenden Ansprüche **dadurch gekennzeichnet, dass** das Tensid PEG-40 Hydrogenated Castor Oil ist.

13. Verwendung einer Zubereitung nach einem der vorangehenden Ansprüche als Haarfestiger.
